# EUROPEAN PATENT SPECIFICATION

(11) **EP 2 429 349 B1**
(45) Date of publication and mention of the grant of the patent: **09.01.2013**
(21) Application number: 10726248.7
(22) Date of filing: 11.05.2010
(51) Int. Cl.: A47G 9/10, A61B 5/00

(54) **AN ACTIVE PILLOW SYSTEM AND A METHOD FOR MANIPULATING A PERSON'S RESTING CONDITIONS**
AKTIVES KISSENSYSTEM UND VERFAHREN ZUR MANIPULATION DER RUHEBEDINGUNGEN EINER PERSON
SYSTÈME D'OREILLER ACTIF ET PROCÉDÉ POUR MANIPULER LES CONDITIONS DE REPOS D'UNE PERSONNE

(30) Priority: 13.05.2009 EP 09160160
(43) Date of publication of application: 21.03.2012
(73) Proprietor: Koninklijke Philips Electronics N.V., 5621 BA Eindhoven (NL)
(72) Inventor: ALBU, Mirela, A., NL-5656 AE Eindhoven (NL); NEWTON, Philip, S., NL-5656 AE Eindhoven (NL); FONSECA, Pedro, NL-5656 AE Eindhoven (NL); CHESTAKOV, Dmitri, NL-5656 AE Eindhoven (NL); DU, Jia, NL-5656 AE Eindhoven (NL); BUIL, Vincentius, P., NL-5656 AE Eindhoven (NL); SCHOLTEN, Liesbeth, M., NL-5656 AE Eindhoven (NL); PRONK, Serverius, P., P., NL-5656 AE Eindhoven (NL); REES, Fiona, NL-5656 AE Eindhoven (NL)
(74) Representative: Kroeze, Johannes Antonius
(86) International application number: PCT/IB2010/052066
(87) International publication number: WO 2010/131190

(56) References cited:
- EP-A1- 1 535 547
- WO-A1-2008/053435
- WO-A1-2010/035198
- US-A1- 2006 050 482

## Description

### FIELD OF THE INVENTION

The present inventions relates to the field of sleep monitoring and active sleep manipulation.

### BACKGROUND OF THE INVENTION

During the summer, because of the high temperature, the pillow of a sleeping person can become very hot and humid from perspiration. Due to this reason many people sleep uncomfortably and sometimes even wake up and turn the pillow to benefit from the colder/dry side.

The state of the art in cooling pillows consists of mostly water based solutions. In most of these solutions the cooling is achieved by filling a hermetic container with water, and placing it in the fridge for a while before use. The container is to be placed afterwards in the pocket of the pillow case and after which the case is slipped over the pillow. By means of the heat exchange process the heat is released it into the air. A great disadvantage of this solution is that the water in the pillow must be changed manually which is very cumbersome. Simultaneously, the low temperature of the cold pack will lead to condensation which will make the pillow wet giving an unpleasant feeling which could actually disturb the sleep more than when the cooling is not used. Further, the prior art document WO 2009 / 005 616 A1 discloses a multiple convective cushion seating and sleeping system incorporating a blower and a stirling cycle to provide temperature modified air which is controllably guided through first and second conductive cushions. Disadvantageously, the integration of a total stirling cycle in a pillow is very difficult, expensive and therefore highly impractically. Another cooling pillow solution disclosed by the patent application WO 00/06006 A1 is based on the principle that the air is made to flow in close contact with the fibrous material being in the vicinity of the body of the user and containing a sufficient amount of water, vaporization of water is promoted, and the head is cooled by absorbing the heat of vaporization. These inventions therefore have an air sending fan, a flow passage through which the air is made to flow, and a vaporization sheet forming the flow passage and containing water. A great disadvantage of this solution is the usage of a fan, which needs a large rotational speed of the rotor to become effective and therefore leads to undesirable noise and vibrations in the pillow.

Currently, on the market, more and more products support people to relax and fall asleep in bed without disturbing their partners. This concept often can be found in different kinds of pillows. For example, pillows with integrated speakers that produce relaxed soothing music and help block environmental noises by creating white noises closely around a person's ears are known. Such products are ideal for private use and travelers due to its portability. There are also pillows with integrated light, which illuminates its surface gradually in the morning to bring a pleasant waking up experiences to a person while keeping the partners asleep. Furthermore, the prior art document WO 2004 / 75 714 A2 discloses a system for manipulating sleep architecture by sub-awakening non-intrusive stimulations based on a real-time, self-adaptive feedback system including a sleep and environment monitoring unit, an integrating, controlling and deciding unit and a stimulation unit. Disadvantageously, this prior art document does not disclose the monitoring of the sleeping person by the aid of an actimetry sensor which provides a very low-cost, compact and accurate determination of the actual sleeping conditions of the sleeping person.

A dehumidifier air passage being capable of providing a sufficient effect of dehumidification is disclosed in EP 1 535 547 A1. The dehumidifier air is used in a dehumidifier for removing moisture by making the outside air flow in the vicinity of the surface of a human body and the like, and comprises a nearly flat spacer and a moisture transmittable sheet. The spacer is used for supporting the moisture transmittable sheet and securing a space through which air flows., and has a nearly flat base and a plurality of long and narrow projections. Each of the projections is formed so that the face of it facing the moisture transmittable sheet is at most 50 % in the area of the face being in contact with the base. A foamed plastic material is used as material for the spacer. The spacer is manufactured by cutting a foamed plastic material or by molding a foamed plastic material.

EP 1 535 547 A1 discloses an active pillow system according to the preamble of claim 1.

### SUMMARY OF THE INVENTION

It is an object of the present invention to provide an active pillow system for manipulating a person's resting conditions which comprises a feed back high-performance and low-noise air conditioner and does not have the drawbacks mentioned in connection with the prior art.

The above mentioned object is accomplished by an active pillow system for manipulating a person's resting conditions comprising:
- a pillow
- a sensor unit and
- an air conditioner unit triggered by the sensor unit,
- wherein the air conditioner unit is integrated into the pillow and characterized in that the air conditioner unit comprises an acoustic synthetic jet cooling mechanism,

Advantageously, the generation of an acoustic synthetic jet with the aid of an acoustic synthetic jet cooling mechanism is much more quiet compared to a fan based water evaporation cooling solution according to the above mentioned state of the art. The usage of the acoustic synthetic jet principle is proposed to control the amount of airflow through air channels in the pillow. The airflow will increase evaporation of sweat that accumulates in the pillow during sleeping or resting of the person. The main effect of the evaporation is to dry the pillow, making it feel more comfortable during the night. In addition as side effect it will help to control the temperature of the pillow.

The acoustic synthetic jet cooling mechanism comprises a diaphragm pump and particularly a loudspeaker, for instance. It is a great advantage of the active pillow system that the acoustic synthetic jet cooling mechanism is able to generate a strong airflow on the one hand, but it does not require a fast rotational movement of a rotor on the other hand. Therefore, aerodynamic noise, vibrations, abrasions and grinding noises caused by the moving rotor can be avoided. This is very important as the active pillow system is provided to improve the person's resting or sleeping conditions and must not be a disturbance source for the resting or sleeping person. In particular, if the person's head lies on the pillow typically with a small distance to the air conditioner. In this active pillow system the air conditioner consists of a loudspeaker in a chamber through a special construction in the chamber a by applying certain frequencies an airjet is produced that can be used to cool devices. It is shown that the acoustic synthetic jet cooling method allows miniaturization, it is very reliable because it has only one moving part that is frictionless driven by a magnetic field. This in contrast to other cooling based methods based on water such as pumps or airflow such as fans.The term "resting" in the sense of the present inventions includes relaxing, as well as sleeping, recovering, regeneration and/or rehabilitation of the person. The wording "pillow" can be understood as a head cushion, a mattress, a sofa cushion, a blanket, a seat cover or the like. Preferably, the acoustic synthetic jet cooling mechanism comprises a membrane which is operated by an electromagnetic and/or a piezoelectric driver.

The sensor unit measures the actual resting conditions of the person. Based on the data collected by the sensor unit the temperature of the pillow is gently adjusted, in particular by a processing unit, to prevent the person to overheat and perspire during hot seasons, for example. Preferably, the sensor unit measures the temperature and/or the humidity of the person and/or the sensor unit measures the temperature and/or the humidity in the environment of the person. Subsequently, the air conditioner is triggered, if required, to improve the climatic conditions to enhance the person's resting conditions. Consequently, the sensor unit incorporates a feedback path for the active pillow system for adjusting optimal resting conditions for the resting person.

In a preferred embodiment the active pillow system according to the present invention comprises a user interface which is wireless connected to the active pillow system, for instance, and allows the resting person to determine individual favored resting conditions. The person can adjust a target temperature value, a target humidity value and/or a target airflow value, for example. Furthermore, the target temperature value, the target humidity value and/or the target airflow value can be choose in dependency of time, in particular the resting time, or in dependency of the sleeping phase. The temperature of the pillow can be lowered during REM (Rapid Eye Movement) phases and increased in the early morning shortly before getting up, for instance.

In a preferred embodiment the sensor unit and/or the air conditioner unit is encased into a cushion material to increase the softness of the surface of the active pillow system to improve the resting comfort. Preferably, the cushion material comprises a memory foam material which provides an individual ergonomic pillow shape.

Furthermore, the active pillow system and/or the sensor unit measures physiological parameters of the person, like pulse rate, electrical activity of the person's brain (EEG), electrical activity of the person's heart (ECG), activation signals of the person's muscles (EMG), galvanic skin response (GSR) and the like. Subsequently, the active pillow system determines the actual resting conditions of the person, like the sleeping depth, the sleeping phase and/or the relaxation level both from the actigraphy measurements and from the physiological parameters to improve the accuracy of the detection.

These and other characteristics, features and advantages of the present invention will become apparent from the following detailed description, taken in conjunction with the accompanying drawing, which illustrates, by way of example, the principles of the invention. The description is given for the sake of example only, without limiting the scope of the invention. The reference figures quoted below refer to the attached drawing.

### BRIEF DESCRIPTION OF THE DRAWINGS

Figure 1 shows an active pillow system according to a first embodiment of the present invention;
Figures 2a, 2b shows a sensor unit of an active pillow system according to the first embodiment of the present invention.

### DETAILED DESCRIPTION OF EMBODIMENTS

The present invention will be described with respect to particular embodiments and with reference to a certain drawing but the invention is not limited thereto but only by the claims. The drawing described is only schematic and is nonlimiting. In the drawing, the size of some of the elements may be exaggerated and not drawn on scale for illustrative purposes.

Where an indefinite or definite article is used when referring to a singular noun, e.g. "a", "an", "the", this includes a plural of that noun unless something else is specifically stated. Furthermore, the terms first, second, third and the like in the description and in the claims are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein. It is to be noticed that the term "comprising", used in the present description and claims, should not be interpreted as being restricted to the means listed thereafter; it does not exclude other elements or steps. Thus, the scope of the expression "a device comprising means A and B" should not be limited to devices consisting only of components A and B. It means that with respect to the present invention, the only relevant components of the device are A and B.

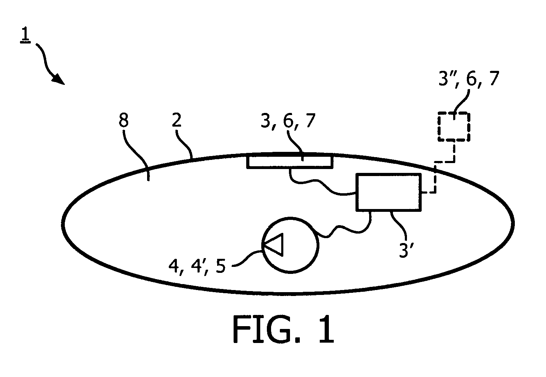

Figure 1 shows a schematic illustration of an active pillow system 1 according to a first embodiment of the present invention. The active pillow system 1 comprises a pillow 2 with a sensor unit 3 and an air conditioner unit 4 inside. The sensor unit 3 measures the actual climatic conditions near the surface of the pillow 2, which is preferably in contact with a head of a person using the pillow 2. The sensor unit 3 incorporates a temperature sensor 6 for measuring the temperature of the head or of the surface of the pillow 2, a humidity sensor for measuring the humidity on the surface of the pillow 2 and/or a galvanic skin response (GSR) sensor 7 for measuring electric conductance of the skin of the person to determine the perspiration of the person. The measured values are used to give an indication about the person's body temperature and humidity and therefore are analyzed by a processing unit 3', which is a part of the sensor unit 3, to determine the actual climatic conditions of the person. In dependency of these determined actual climatic conditions the air conditioner unit 4 is triggered by the processing unit 3' to provide optimal climatic conditions to the person. If the temperature of the pillow 2 or of the head of the person exceeds a certain threshold, the air conditioner 4 starts to cool down the surface of the pillow 2 by airflow and if the temperature falls below another threshold, the air conditioner 4 stops cooling, for instance. Therefore, the air conditioner 4 comprises an acoustic jet cooling mechanism 4', wherein the airflow is provided by an oscillating membrane and not by a rotating impeller. In this active pillow system 1 the air conditioner 4 consists of a loudspeaker 5 in a chamber through a special construction in the chamber a by applying certain frequencies an airjet is produced that can be used to cool devices. The noise produced by such a system is less than a regular fan based solution and could be further reduced by sound proofing the device. In this study it is shown that the acoustic synthetic jet cooling method allows miniaturization, it is very reliable because it has only one moving part that is frictionless driven by a magnetic field. This in contrast to other cooling based methods based on water such as pumps or airflow such as fans.

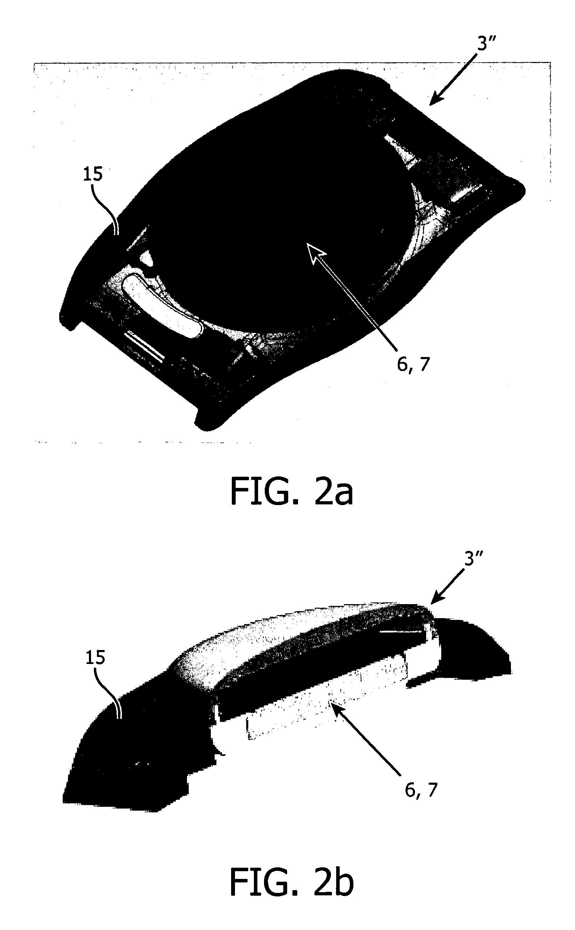

In another preferred embodiment the processing unit 3' is wireless connected to a further sensor unit 3" which is directly attached to the body of the person by a wristband of a wrist watch. The further sensor unit 3" comprises a temperature sensor 6, a galvanic skin sensor 7 and a pulse sensor. The sensor unit 3" is integrated into the housing 15 of a wrist watch, for example, which straight contacts the skin of the person. The housing 15 of the wrist watch and the further sensor unit 3" are schematically illustrated in figures 2a and 2b.

## Claims

1. An active pillow system (1) for manipulating a person's resting conditions comprising:
- a pillow (2)
- a sensor unit (3) and
- an air conditioner (4) unit triggered by the sensor unit (3),
- wherein the air conditioner unit (4) is integrated into the pillow (2), **characterized in that** the air conditioner unit (4) comprises an acoustic synthetic jet cooling mechanism (4').

2. The active pillow system (1) according to claim 1, **characterized in that** the synthetic jet cooling mechanism (4') comprises a loudspeaker (5) and/or a diaphragm pump.

3. The active pillow system (1) according to one of the claims 1 or 2, **characterized in that** the sensor unit (3) comprises a humidity sensor, a temperature sensor (6) and/or a galvanic skin response sensor (7).

4. The active pillow system (1) according to one of the preceding claims, **characterized in that** the sensor unit (3) and/or the air conditioner unit (4) are encased into a cushion material (8) of the pillow (2), in particular a foam material.

5. The active pillow system (1) according to claim 4, characterized that a interlayer is assembled between the cushion material (8) on the one hand and the sensor unit (3) and/or the air conditioner unit (4) on the other hand, wherein the cushion material is more flexible than the interlayer.

6. The active pillow system (1) according to one of the claims 3 or 4, **characterized in that** the foam material and/or the interlayer comprise multiple air ventilation ducts.

## Patentansprüche

1. Aktives Kissensystem (1) zur Manipulation der Ruhebedingungen einer Person, das Folgendes umfasst:
- ein Kissen (2)
- eine Sensoreinheit (3) und
- eine durch die Sensoreinheit (3) ausgelöste Luftaufbereitungseinheit (4),
- wobei die Luftaufbereitungseinheit (4) in das Kissen (2) integriert ist,
**dadurch gekennzeichnet, dass** die Luftaufbereitungseinheit (4) einen akustischen synthetischen Strahlkühlungsmechanismus (4') umfasst.

2. Aktives Kissensystem (1) nach Anspruch 1, **dadurch gekennzeichnet, dass** der synthetische Strahlkühlungsmechanismus (4') einen Lautsprecher (5) und/oder eine Membranpumpe umfasst.

3. Aktives Kissensystem (1) nach einem der Ansprüche 1 oder 2, **dadurch gekennzeichnet, dass** die Sensoreinheit (3) einen Feuchtigkeitssensor, einen Temperatursensor (6) und/oder einen galvanischen Hautreaktionssensor (7) umfasst.

4. Aktives Kissensystem (1) nach einem der vorhergehenden Ansprüche, **dadurch gekennzeichnet, dass** die Sensoreinheit (3) und/oder die Luftaufbereitungseinheit (4) in einem Polstermaterial (8) des Kissens (2) eingeschlossen sind, insbesondere in einem Schaumstoff.

5. Aktives Kissensystem (1) nach Anspruch 4, **dadurch gekennzeichnet, dass** eine Zwischenschicht zwischen dem Polstermaterial (8) auf der einen Seite und der Sensoreinheit (3) und/oder der Luftaufbereitungseinheit (4) auf der anderen Seite eingebaut ist, wobei das Polstermaterial flexibler ist als die Zwischenschicht.

6. Aktives Kissensystem (1) nach einem der Ansprüche 3 oder 4, **dadurch gekennzeichnet, dass** der Schaumstoff und/oder die Zwischenschicht mehrere Luftventilationskanäle umfassen.

## Revendications

1. Système d'oreiller actif (1) pour manipuler les conditions de repos d'une personne, comprenant :
- un oreiller (2),
- une unité de détection (3), et
- une unité de climatisation (4) déclenchée par l'unité de détection (3),
- dans lequel l'unité de climatisation (4) est intégrée à l'oreiller (2),
**caractérisé en ce que** l'unité de climatisation (4) comprend un mécanisme de refroidissement par jet synthétique acoustique (4').

2. Système d'oreiller actif (1) selon la revendication 1, **caractérisé en ce que** le mécanisme de refroidissement par jet synthétique (4') comprend un haut-parleur (5) et/ou une pompe à diaphragme.

3. Système d'oreiller actif (1) selon la revendication 1 ou 2, **caractérisé en ce que** l'unité de détection (3) comprend un capteur d'humidité, un capteur de température (6) et/ou un capteur de réponse cutanée galvanique (7).

4. Système d'oreiller actif (1) selon l'une quelconque des revendications précédentes, **caractérisé en ce que** l'unité de détection (3) et/ou l'unité de climatisation (4) sont encastrées dans un matériau de coussin (8) de l'oreiller (2), en particulier un matériau de mousse.

5. Système d'oreiller actif (1) selon la revendication 4, **caractérisé en ce qu'**une couche intermédiaire est assemblée entre le matériau de coussin (8) d'une part et l'unité de détection (3) et/ou l'unité de climatisation (4) d'autre part, dans lequel le matériau de coussin est plus souple que la couche intermédiaire.

6. Système d'oreiller actif (1) selon la revendication 3 ou 4, **caractérisé en ce que** le matériau de mousse et/ou la couche intermédiaire comprennent plusieurs conduits de ventilation d'air.
